# EUROPEAN PATENT APPLICATION

(11) **EP 1 369 692 A2**
(43) Date of publication of application: **10.12.2003**
(21) Application number: 03447140.9
(22) Date of filing: 04.06.2003
(51) Int. Cl.: G01N 33/543

(54) **Sensor surface**

(30) Priority: 25.10.2002 EP 02447203; 19.09.2002 US 412100 P; 04.06.2002 US 385869 P
(71) Applicant: Interuniversitaire Microelectronica Centrum vzw ( IMEC), 3001 Leuven (BE)
(72) Inventor: Frederix, Filip, 3511 Hasselt (BE)
(74) Representative: Van Malderen, Joelle

(57) **Abstract**

The present invention is related to a device suitable for the preparation of a sensor, comprising
- a substrate comprising a metal layer, said metal layer comprising at least a first region wherein to a first region is attached a first species having the chemical formula:

   X-R₁-S-S-R₂-Y
wherein R₁ and R₂ represent independently from each other spacer of n carbon atoms, n being an integer higher than 11;
wherein X represents 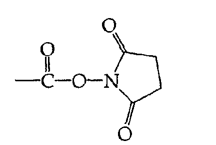 and Y represents an organic group. The first species is able to form a self-asssembling monolayer on said first region.

## Description

### Field of the invention

The present invention is related to an electronic sensor device and in particular to devices comprising self-assembled monolayers (SAM) bound to a recognition molecule to perform highly sensitive and selective analysis. Furthermore the present invention is related to a method for the preparation of such monolayers and corresponding sensors.

### Background of the invention

Health and environment related fields, faces various biochemical processes which have to be evaluated rapidly at decreasing detection levels. Many biochemical analytical methods involve immobilisation of a biological molecule on a surface.

The increasing miniaturisation and the demand for sensitivity require a covalent immobilisation of biomolecules. Affinity biosensor transducers are defined as systems containing at least one biological element able to recognise an analyte. This element is called the biological recognition layer.

The biological recognition layer consists of a probe molecule, covalently bound to a linking layer, which makes the connection with the transducer. The concentration of this analyte is translated by an electrical signal via the right combination of an efficient biological recogniser and an adequate translation system.

Such sensors combine the extremely high biochemical selectivity with the speed of micro-electronic transducers.

The presence of analytes can be detected by sensing fluids using acoustic waves as described in US4361026. The solid phase refers to any material insoluble in a medium containing a target molecule. The substrate can be a deposit of a metal film on any convenient support or any other solid surface able to selectively bind monolayers. Possible metals are: gold, silver, GaAs, palladium, platina, copper, and the like.

Silanes and alkyl phosphate monolayers can also be used on oxide material supports like SiO₂, Nb₂O₅, TiO₂, ZrO₂, Al₂O₃, Ta₂O₅.

A biosensor must respond to major qualities like stability, specificity, selectivity and reproducibility.

For all those reasons, affinity biosensors are not yet commercially available. The major challenge consists of the realisation of new specific and selective self-assembled monolayers and the receptors. An analyte must be detectable in an excess of other proteins.

The most common receptors are antibodies and specific binding proteins which have a reversible specific binding affinity for an analyte. Chemical modifications of the surface moieties may create new surface functionalities, such as, for example, amine-terminated functional groups, appropriate for particular diagnostic or therapeutic operations.

Bamdad et al in US Patent Nos. 5,620,850 and 6,127,129 discloses a biosensor of a formula X-R-Ch-M adhered to a surface as part of a self assembled monolayer, where X is a functionality that adheres to the surface, R is a spacer moiety and Ch is a chelating agent for the metal ion M. The monolayers described in this patent only have limited surface accessibility for biological binding and oriented immobilisation. Moreover, the monolayers can only be achieved via an extra crosslinker step.

Lahiri et al. (Anal. Chem., 1999, 71, 777-790) describe a method for immobilising proteins on mixed self-assembling monolayers of alkanathiols. The method comprises the steps of obtaining a N-hydroxysuccinimidyl (NHS) ester from the carboxylic acid groups of the self-assembling monolayer and coupling this ester to a free amine group of the protein. In a first step, a self-assembling monolayer is formed on a gold surface. The self-assembling monolayer has free carboxylic acid groups. In a next step, the surface carboxylic acid groups are activated with NHS and EDC (ethylene dichloride) to form the NHS ester and displacement of the NHS ester with an amino group of the protein to form an amide bond. Since several steps have to be performed after deposition of the SAM on the substrate, the yield reduces after each step, resulting in a lower yield of the immobilisation degree.

Dojindo (Japanese company, see for instance http://www.dojindo.com) discloses succinimidyl alkane disulfide compounds such as dithiobis (succinimidyl octanoate) in generic forms without precise application data.

### Aims of the invention

The aim of the present invention is to provide a sensing device comprising a self-assembling monolayer (SAM) suitable for the fabrication of a high selectivity, high stability and high reproducibility sensor, linked to a recognition molecule.

A supplementary aim of the present invention is to provide a method for producing such a device.

It is a further aim of the invention to provide a compound suitable for forming said monolayer.

### Description of the figures

Figure 1 represents 16,16'-Dithiohexadecanoic acid di(N-hydroxysuccinimide ester) deposited on a substrate having a gold layer.

Figure 2 represents a Grazing Angle FTIR of 16,16'-Dithiohexadecanoic acid di(N-hydroxysuccinimide ester) deposited on substrate having a gold layer.

Figure 3 represents the molecules used for the comparative tests deposited on a substrate having a gold layer.
a: 16-mercapt-1-hexadecanoic acid
b: 11-mercapto-1-undecanol and 16-mercapto-1-hexadecanoic acid
c: 16-mercapto-1-hexadecanoic acid and 2-(2-(2-(6-Mercapto hexyoxy) ethoxy) ethoxy) ethanol (6-PEO)

Figure 4 represents the detected concentration of human transferine for different types of monolayers versus RIU (refractive index units).

Figure 5 represents a mixed monolayer of 16,16'-Dithiohexadecanoic acid di(N-hydroxysuccinimide ester) and 10,10'dithioundecanol) on a gold substrate.

Figure 6 represents an interdigitated electrode configuration suitable for the fabrication of a sensor.

Figure 7 represents the chemical structure of another chemical compound.

Figure 8 represents chemical compounds to be used together with the molecule represented in figure 7 (mixed monolayers)

Figure 9 represents anti-human transferrin immobilized on DSH SAM. 91-94 (Experiments 1 to 4) refer to experiments performed at different times.

Figure 10 represents DSH SAM's reacting with antibodies (step 101) and blocked with a blocking agent (step 102).

Figure 11 represents the recognition of a specific analyte HT and a non-specific analyte IgG on a surface with Streptavidin immobilisation.

### Summary of the invention

The present invention discloses a device suitable for the preparation of a sensor, comprising
- a substrate comprising a metal layer, said metal layer comprising at least a first region wherein to said first region is attached a first species having the chemical formula:

   X-R₁-S-S-R₂-Y
wherein R₁ and R₂ represent independently from each other a spacer of n carbon atoms, n being an integer higher than 12;
wherein X represents 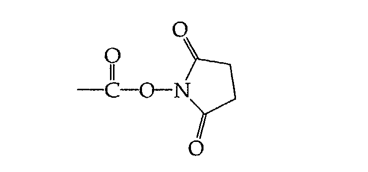 and Y represents an organic group. Said first species forms a self-assembling monolayer on said first region.

In a further embodiment of the present invention n is an integer comprised between 13 and 30.

In a more particular embodiment of the present invention, said spacer is interrupted by at least one or q heteroatoms. Said heteroatom can be oxygen.

In an additional embodiment of the present invention, R₁ and R₂ are independently from each other a hydrocarbon chain. In a more particular embodiment, R₁ and R₂ are an alkyl chain (CH₂)ₙ.

In another embodiment, R₁ and R₂ are independently from each other Q-R, Q being a hydrocarbon group and Q being bound to the sulphur atom, R being a chemical group for avoiding non-specific adsorption, while Q is for forming the self-assembling monolayer.

In another embodiment, R₁ and R₂ are independently from each other (CH₂)ₐ - (CH₂-CH₂-O)_{b}- (CH₂)_{c}, a being an integer, b being an integer and c being an integer. Preferably, a is comprised between 1 and 20, b is comprised between 1 and 10 and is an comprised integer between 1 and 3.

In a further embodiment, R₁ and R₂ have the same chemical composition.

In a further embodiment of the present invention Y comprises a chemical group selected from the groups consisting of carboxyl, hydroxyl, cyano, amine, epoxy and vinyl groups.

In another particular embodiment of the present invention Y is

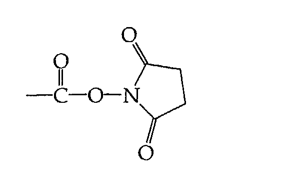

In an additional particular embodiment of the present invention said first species is 16,16'-dithiohexadecanoic acid di(N-hydroxysuccinimide ester).

In an additional particular embodiment, said first species is

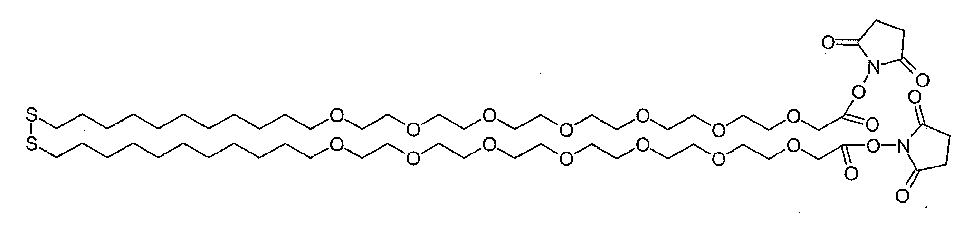

In a further embodiment of this invention, said metal layer further comprises a second region, wherein to said second region is attached a second species having the chemical formula:

W-R₃-S-S-R₄-Z,

wherein R₃ and R₄ represent independently from each a spacer, optionally interrupted by a heteroatom, W and Z being organic groups, and wherein said first species and said second species form a mixed self-assembled monolayer on the metal layer.

In an additional embodiment of the invention, R₃ and R₄ are a spacer of m carbon atoms, optionally interrupted by q heteroatoms wherein m or (m+q) is an integer higher than 6.

In a further additional embodiment of the invention, W and Z are independently from each other selected from the group consisting of carboxyl, hydroxyl, cyano, amine, epoxy and vinyl groups.

In a general embodiment of the invention, said metal is selected from the groups consisting of gold, silver, mercury, aluminium, platinum, palladium, copper or alloys thereof.

In a general embodiment of the present invention m or (m + q) are lower than n or (n+q).

Another aspect of the present invention discloses a method for producing a device, suitable for determining the presence of a target molecule, comprising the steps of:
- providing a substrate comprising at least a metal layer,
- providing a species having the formula

   X-R₁-S-S-R₂-Y

   wherein R₁ and R₂ represent independently from each other a spacer of n carbon atoms, n being an integer higher than 12
   wherein X represents 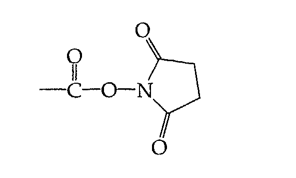 and wherein Y is an organic group, and
- subjecting said substrate to said species to form a self-assembled monolayer on said substrate.

An additional embodiment further comprises the step of covalently binding a recognition molecule to the X-group of said self-assembling monolayer such that said first species becomes NH₂-CO-R₁-S-S-R₂-Y.

Another embodiment further comprises the step of covalently binding a recognition molecule to at least one of the X-group or the Y group of said self-assembling monolayer.

In a particular embodiment, said recognition molecule is a chemical compound with a free NH₂ group and/or a recognition molecule selected from the group consisting of antigen, antibody, nucleic acid strand, hormones, enzymes, and polyaminoacids.

Another key embodiment of the present invention discloses a method further comprising the step of:
- providing a second species, said second species being different from said first species,
- subjecting said substrate to said second species to form a mixed self-assembling monolayer on said metal layer.

In a particular embodiment said second species has the chemical formula:

W-R₃-S-S-R₄-Z,

wherein R₃ and R₄ represent independently from each other a spacer, optionally interrupted by a heteroatom, W and Z being organic groups.

Finally, the present invention discloses a compound, suitable for forming a monolayer on a sensor device, having the formula:

X-R₁-S-S-R₂-Y

wherein R₁ and R₂ represent independently from each other a spacer of n carbon atoms, n being an integer higher than 12 wherein X represents 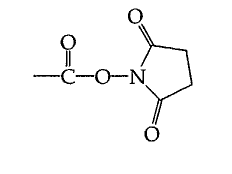 and wherein Y is an organic group.

In a further embodiment of the present invention n is an integer comprised between 13 and 30.

In a more particular embodiment of the present invention, said spacer is interrupted by at least one heteroatom. Said heteroatom can oxygen.

In an additional embodiment of the present invention, R₁ and R₂ are independently from each other a hydrocarbon chain. In a more particular embodiment, R₁ and R₂ are an alkyl chain (CH₂)ₙ.

In another embodiment, R₁ and R₂ are independently from each other Q-R, Q being a hydrocarbon group and Q being bound to the sulphur atom, R being a chemical group for avoiding non-specific adsorption, while Q is for forming the self-assembling monolayer.

In another embodiment, R₁ and R₂ are independently from each other (CH₂)ₐ- (CH₂-CH₂-O)_{b}- (CH₂)_{c}, a being an integer, b being an integer and c being an integer. Preferably, a is comprised between 1 and 20, b is comprised between 1 and 10 and is an comprised integer between 1 and 3.

In a particular embodiment of the present invention R₁ and R2 have the same chemical composition.

In another particular embodiment of the present invention Y is a chemical group selected from the group consisting of carboxyl, hydroxyl, cyano, amine, epoxy and vinyl groups.

In an additional particular embodiment of the present invention X and Y have the same chemical composition.

In a specific embodiment of the present invention said monolayer is 16,16'-dithiohexadecanoic acid di(N-hydroxysuccinimide ester).

In an additional particular embodiment, said first species is

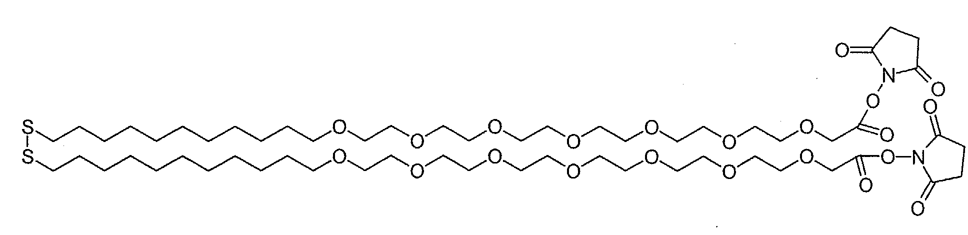

Finally the use of a compound is disclosed for the preparation of a self-assembling monolayer on a substrate of a sensor device.

### Detailed description of the invention

In a first aspect of the present invention, a compound, suitable for the fabrication of a self-assembling monolayer, is disclosed. The compound has the chemical formula :

X-R₁-S-S-R₂-Y

wherein R₁ and R₂ represent independently from each other a spacer of n carbon atoms, wherein n is an integer higher than 11;
wherein X represents 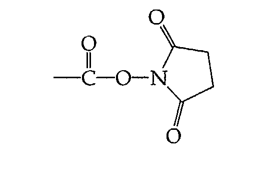 and Y represents an organic group. Said compound allows the formation of a self-assembled monolayer.

For the purpose of this invention the group 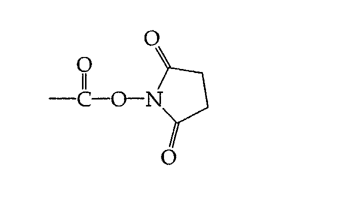 is also referred to as NHS.

Self-assembled monolayers should be considered as a relative ordered assembly of molecules that spontaneous attach (also called chemisorb) on a surface. The molecules are preferably oriented parallel and preferably under an angle of at least 45 degrees to the surface.

Each group being part of a self-assembling monolayer preferably contains a functional group for attaching to the surface (53) and a functional group that binds to the recognition molecule (35,55). In the present invention, the functional group being able to attach to the surface is the disulphide group -S-S- and the functional group being able to bind a recognition molecule is the NHS group.

The functional group -S-S- is able to adhere (chemisorb) to a surface such as a metal and can chemically interact with the metal surface (12,52). The interaction between the sulphur atom and the substrate is known to people skilled in the art and is described in Nuzzo, R.G.; Allara, D.L. ; J. Am. Chem. Soc. 1983, 105, 4481, and Abraham Ulman, An Introduction to Ultra thin Organic Films, Academic Press Inc, 1991.

The NHS group (55) can be used for surface immobilisation of a recognition molecule. The recognition molecules can be bound to this group and in particular to the NH₂ group of a recognition molecule.

The NHS group is highly reactive towards thiol groups and a chemical compound comprising a thiol would react with the NHS group. Therefore, a disulphide is required such that binding between the NHS group and the sulphur atoms is prevented.

Y can be a chemical group selected from the group consisting of carboxyl, hydroxyl, cyano, amine, epoxy and vinyl groups. In a preferred embodiment, Y is

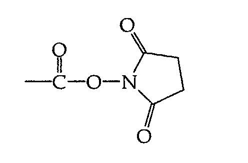

R₁ and R₂ represent independently from each other a spacer (13,31,32,33,34) of n carbon atoms, wherein n is an integer higher than 6 and preferably 11. Said spacer promotes the formation of a self-assembling monolayer and can be a hydrocarbon chain. "Hydrocarbon" chain can be understood as including an alkyl, alkenyl, alkynyl, cyclic alkyl, aryl, alkyl bound to aryl, alkenyl bound to aryl and alkynyl bound to aryl. In an embodiment, the spacer is an alkane. Said spacer could also represent a hydrocarbon interrupted by a -CO-(ketone), -CONH-, -CONHCO-, -CSNH-, -CS-, and the like. The hydrocarbon chain can also be branched. The heteroatom could be selected from the group consisting of -NH-, -O-, -S-, -CS-. In particular, the heteroatom could be O. The spacer could comprise a first part which is a hydrocarbon chain and a second part which is a a hydrocarbon chain interupted by a heteroatom such as oxygen. R₁ and R₂ can have the same chemical composition such that a symmetrical molecule is formed. Symmetrical molecules have generally the advantage of a more straightforward synthesis.

In an embodiment of the invention, n is an integer. In a preferred embodiment n is higher than 4, higher than 6, higher than 8, higher than 10, higher than 11, higher than 12, higher than 13, higher than 15 or higher than 20. Alternatively, n is comprised between 12 and 30, between 13 and 30, between 12 and 25, between 12 and 30, between 13 and 30 or between 12 and 22. Preferably, n is between 13 and 25. Most preferably, n is 16.

In a particular embodiment, R₁ and R₂ are independently from each other a spacer comprising two parts, a first part for obtaining a stable ordered monolayer and a second part for avoiding non-specific adsorption. In a particular embodiment, R₁ and R₂ are independently from each other (CH₂)ₐ - (CH₂-CH₂-O)_{b}- (CH₂)_{c}, a being an integer, b being an integer and c being an integer. The alkyl chain is to achieve a stable ordered and reproducible system while the polyethyleneoxide groups are for avoiding non-specific adsorption. a can be comprised between 1 and 20, between 6 and 20, between 6 and 15. b is comprised between 1 and 10, between 1 and 8. c is an integer between 1 and 3, c can be 1, 2 or 3. The total number of carbon atoms n is preferably higher than 11, higher than 12, higher than 15, higher than 22, higher than 25.

In a particularly preferred embodiment, said chemical compound is 16,16'-dithiohexadecanoic acid di(N-hydroxysuccinimide ester), with molecular formula: C₂₇H₄₂N₂O₈S₂ and molecular weight: 586.

Said chemical compound can be characterised as follows:
Mass spectroscopy: molecular weight: 769.12
H NMR: δ 2.83 singlet, 2.68 triplet, 2.60 triplet, 1.78-1.63 multiplet, 1.43-1.29 multiplet.

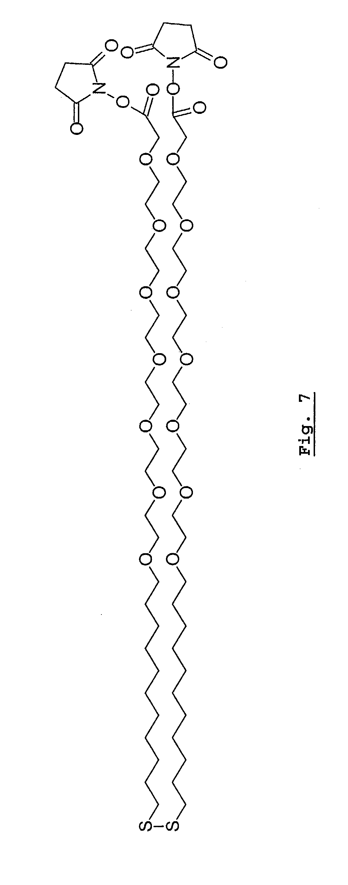

In another particular embodiment, said chemical compound is the chemical compound as disclosed in figure 7 with the molecular formula C₅₈H₁₀₄N₂O₂₂S₂ and molecular weight : 1245,58.

In a second aspect of this invention, a device, suitable for the fabrication of a sensor, is disclosed. Said device comprises:
- a substrate comprising a metal layer, said metal layer comprising at least two regions, wherein to a first region is attached a first species, said first species being the compound disclosed in the first aspect of the invention,
wherein to a second region is attached a second species having the chemical formula:

W-R₃-S-S-R₄-Z,

wherein W and Z represent organic groups, R₃ and R₄ represent independently from each other spacer, optionally interrupted by a heteroatom. Said first species and said second species are selected such that a mixed self-assembled monolayer is formed on the metal layer. A mixed self-assembled monolayer results in better sensitivity of the recognition molecule towards the target molecule in the medium. Certain species are used to prevent non-specific adsorption.

The molar ratio of the second species or the first species can be 1000:1, 500:1, 100:1, 80:1, 70:1, 60:1, 50:1, 20:1, 10:1, 5:1, 95:5, 90:10, 80:20, 70:30 or 60:40. The final ratio of the second and the first species can be determined by spectroscopic techniques available to a person skilled in the art.

A mixed monolayer is desired as it results in a better sensitivity of the recognition molecule to the target molecule in the medium. Non-specific adsorption should be avoided when the device is used as a sensor. Non-specific adsorption refers to interaction between the recognition molecule immobilised at the surface and any species being present in a medium that preferably contains the target molecule. "Any species" excludes the target molecule.

The second species can have the chemical formula W-R₃-S-S-R₄-Z, wherein R₃ and R₄ represent independently a spacer of m carbon atoms optionally interrupted by q heteroatoms and wherein m or (m + q) being an integer. Preferably, m or (m + q) are lower than n or (n + p).

The spacer promotes the formation of a self-assembling monolayer. The spacer can be a hydrocarbon chain. Hydrocarbon chain can be understood as including an alkyl, alkenyl, alkynyl, cyclic alkyl, aryl, alkyl bound to aryl, alkenyl bound to aryl, alkynyl bound to aryl and can also represent a hydrocarbon interrupted by a -CO-(ketone), -CONH-, -CONHCO-, -CSNH-, -CS-, and the like. The hydrocarbon chain can be branched. The heteroatom can be selected from the group consisting of -NH-, -O-, -S-, -CS-. R₃ and R₄ can have the same chemical composition such that a symmetrical molecule is formed. Symmetrical molecules generally have the advantage of a more straightforward synthesis.

In an embodiment, R₃ and R₄ are independently a spacer of m carbon atoms, optionally interrupted by q heteroatoms wherein (m+q) or m is an integer higher than 6. In a preferred embodiment, R₃ and R₄ are independently selected from the group consisting of an alkyl chain (CH₂)ₘ (81) with m an integer higher than 6 and an alkyl chain interrupted by q heteroatom with (m + q) higher than 6. W and Z are independently selected from the group consisting of carboxyl, hydroxyl, cyano, amine, epoxy and vinyl groups.

In another embodiment, R₃ and R₄ are independently from each other a spacer comprising two parts, a first part for obtaining a stable ordered monolayer (82) and a second part for avoiding non-specific adsorption (83). In a particular embodiment, R₃ and R₄ are independently from each other (CH₂)ₑ- (CH₂-CH₂-O) _{f}- (CH₂)_{g}, e being an integer, f being an integer and g being an integer. The alkyl chain is to achieve a stable ordered and reproducible system while the polyethyleneoxide groups are for avoiding non-specific adsorption. e can be comprised between 1 and 20, between 5 and 20, between 5 and 15, between 5 and 12, 6 or 11. f is comprised between 1 and 10, between 1 and 8, between 2 and 6, 3, 4 or 5. g is an integer between 1 and 3, c can be 1, 2 or 3. The total number of carbon atoms n is preferably higher than 3, higher than 6, higher than 8 higher than 10.

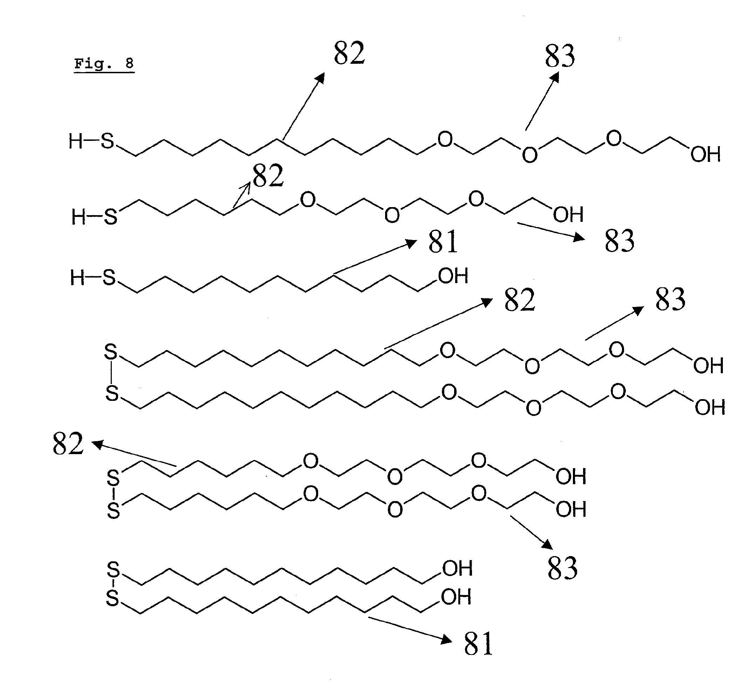

Examples of the second species are represented in figure 8.

In a preferred embodiment, disulphides are used, such that the reaction between the sulphur atoms of the second species and the NHS group of the first species is avoided during the deposition of the mixed monolayer.
W and Z are organic groups selected such that non-specific adsorption is reduced. The groups W and Z should have a functionality that does not adhere to the target molecule in the medium and that does not attach to the surface.

Particularly, W and Z could bear an OH or a sugar moiety.

In a preferred embodiment, said second species has the chemical formula HO-(CH₂)ₘ-S-S-(CH₂)ₘ-OH, m being an integer higher than 4, higher than 7, preferably m being equal to 10.

In another particular embodiment, the molecules are referred to in figure 8 are disclosed.

Said first species and said second species can be selected such that they are able to be deposited from a solution or a mist on the required (first or second) part of the substrate. Said substrate preferably contains a metal layer such as, but not limited hereto, gold, silver, mercury, aluminium, platinum, palladium, copper, cadmium, lead, iron, chromium, manganese, tungsten and alloys thereof.

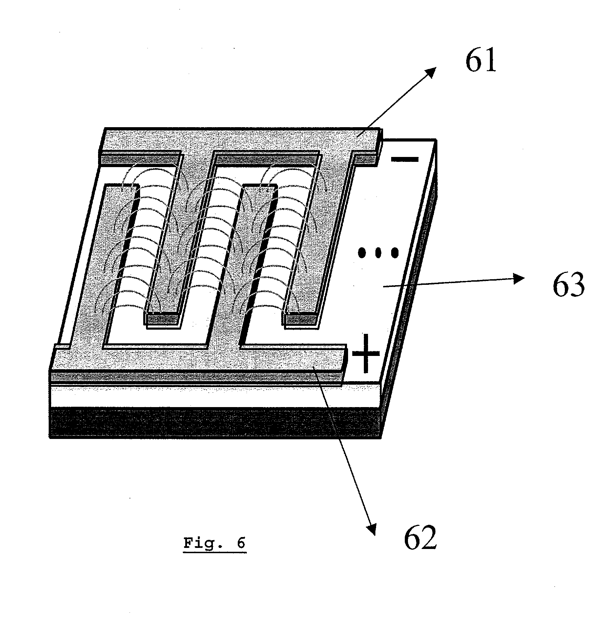

According to one of the preferred embodiments, a combination of gold as surface material and the first and second species as described in this invention is selected. Said metal layer can, but does not necessarily, cover the whole substrate. Said metal layer can be finger-shaped such as interdigitated electrodes (see figure 6).

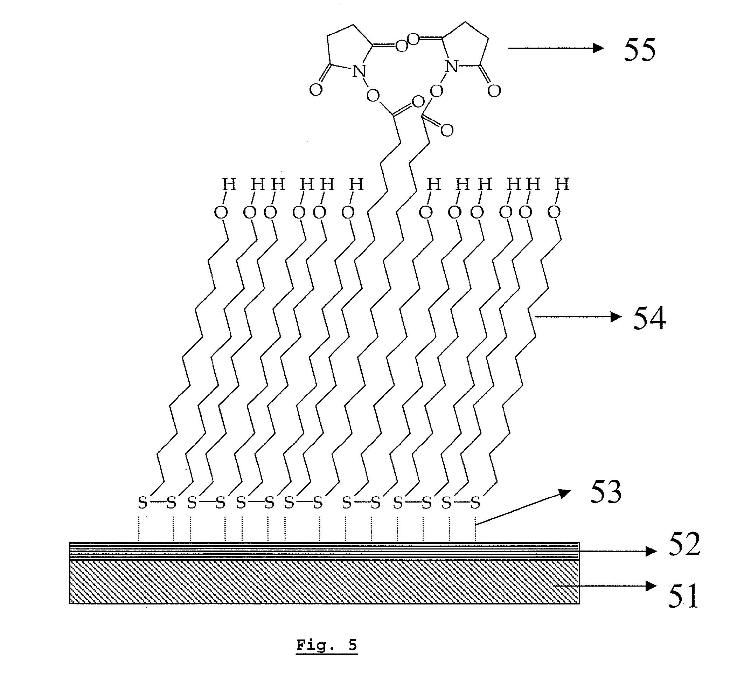

In another preferred embodiment, a device as represented in figure 5 is disclosed. Said device comprises a substrate having a gold surface. A mixed monolayer is chemisorbed (53) to the gold layer (52) deposited on a substrate (51). The mixed monolayer comprises two species, 16,16'-dithiohexadecanoic acid di(N-hydroxysuccinimide ester) (55) and HO-(CH2)₁₁-S-S-(CH₂)11-OH (54) in a 70:30 ratio.

The NHS group in the first species as described in the first aspect of the invention is able to bind a NH₂- group of the recognition molecule (NH₂-B) such that a O=C-NH-R group is formed.

Preferably, the first species and the second species are dissolved in the same solvent and both species are subjected together to the substrate. The solvent should be essentially free of water.

In second aspect of this invention, a device, suitable for the fabrication of a sensor is disclosed. The device comprises :
- a substrate comprising a metal layer, said metal layer comprising at least a first region wherein to a first region is attached a first species having the chemical formula:

   X-R₁-S-S-R₂-Y
wherein R₁ and R₂ represent independently from each other a spacer of n carbon atoms, wherein n is an integer higher than 12;
wherein X represents 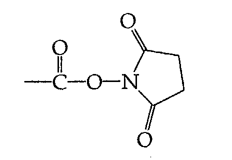 and Y represents an organic group. Said first species can be characterized by the properties described in the first aspect of this invention.

In an embodiment, the metal layer of the device further comprises a second region, wherein to said second region is attached a second species having the chemical formula:

W-R₃-S-S-R₄-Z,

wherein R₃ and R₄ represent independently from each a spacer, optionally interrupted by a heteroatom, W and Z being organic groups, and wherein said first species and said second species forms a mixed self-assembled monolayer on the metal layer. Said second species can be characterized by the properties described in the first aspect of this invention.

Said metal is selected from the groups consisting of gold, silver, mercury, aluminium, platinum, palladium, copper or alloys thereof.

In a third aspect of this invention, a sensor is disclosed. The chemical nature of the self assembling monolayer used in the sensor is different of the one used in the device in such a way that the chemical group of the SAM has a sensing or a recognition function towards a chemical compound belonging to the external medium (target molecule to be analysed) said sensor comprises:
- a substrate comprising a metal layer, said metal layer comprising at least two regions,
wherein to a first region is attached a further species, said further species having the chemical formula:

Y-R₁-S-S-R₂-CO-NH-B

wherein B is part of a recognition molecule NH₂-B.

NH₂-B is a recognition molecule, i.e a molecule able to selectively interact with a target molecule that is present in an external medium.

Besides recognition molecules with a terminal -NH₂ group, other groups can be bound to the succinimide group. This surface synthesis requires an extra step, wherein a cross-linker molecule is bound to the NHS group and the recognition molecule.

Said recognition molecule can be, but is not limited hereto, a molecule, wherein the -NH₂ group is covalently bound to the C=O group of the NHS of the monolayer, already deposited on the substrate. Said recognition molecule could be, but is not limited hereto, a nucleic acid strand (DNA, PNA, RNA), hormones, antibiotics, antibodies, antigens, enzymes, drugs or drugs of abuse or molecules such as recognition molecules for gases or ions.

According to a preferred embodiment, the sensor is suitable for determining the presence of a compound, such as a target molecule in a medium. Target molecule should be understood as a chemical compound that is able to interact with said recognition molecule. The target molecule could be, but is not limited hereto, complementary nucleic acid strand (DNA, PNA, RNA), hormones, antibiotics, antibodies, antigens, enzymes, drugs or drugs of abuse or molecules such as specific molecules present in for gases or ions. The sensor can be arranged such that it acts as a biosensor chip (SPR chip, SAW chip, ...).

The device and the sensor can further comprise a transducer. The self-assembling monolayer is deposited on the metal surface of the transducer. The transducer can be part of, but is not limited hereto, a Surface Plasmon Resonance sensor, Surface Acoustic Wave sensors, Quartz Crystal Microbalance, Amperometric sensors, Capacitive sensors, Interdigitated Electrodes or ChemFET sensors. Figure 6 represents a sensor comprising interdigitated electrodes as transducer, wherein 61 is the negative electrode, 62 is the positive electrode and 63 is a substrate on which the electrodes are deposited. The monolayer as described in this invention can be deposited on the electrodes.

In a fourth aspect of the invention, a method for producing a device, suitable for determining the presence of a compound is disclosed. Said method comprises the steps of:
- providing a substrate comprising at least a metal layer,
- providing a first species having the formula

   X-R₁-S-S-R₂-Y
wherein R₁ and R₂ represent independently from each other a spacer of n carbons, , wherein n is an integer higher than 11 or higher than 12, wherein X represents 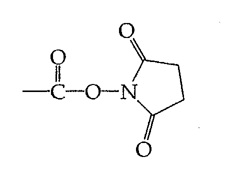 and wherein Y is an organic group,
- and subjecting said substrate to said first species to form a self-assembled monolayer on said substrate.

Said spacer of n carbon atoms can optionally be interrupted by p heteroatoms.

Said first species can have the characteristics as described in the first and second aspect of the invention.

Contrary to the prior art, the NHS terminated molecule can be directly deposited on the substrate without any intermediate step. This results in a higher yield.

Said method can further comprise the step of covalently binding a recognition molecule NH₂-B to said X group of said self-assembling monolayer. The NH₂ group of NH₂-B will react with the NHS group of the first species such that a CO-NH-B group is formed. In further step, substrate can be subjected to a blocking agent such that the unreacted NHS groups are deactivated. Consequently, interaction between the target molecule and the unreacted NHS groups are substantially avoided.

In a preferred embodiment, the method further comprises the step of:
- providing a second species, said second species being different from said first species,
- subjecting said substrate to said second species such that a mixed self-assembling monolayer is formed on said metal layer.

Said second species can have the chemical formula and function as described in the first, second or third aspects of the invention.

Preferably, the step of subjecting the substrate to a first species and subjecting the substrate to a second species are performed together, i.e. the first species and the second species are dissolved in the same solvent and that both species are subjected together to the substrate. The solvent should be essentially free of water.

Said method can further comprise the step of covalently binding a recognition molecule NH₂-B to said X group of said self-assembling monolayer. The NH₂ group of NH₂-B will react with the NHS group of the first species such that a CO-NH-B group is formed. The interaction between the W and Z group of the second species and the recognition molecule is preferably as low as possible, there is preferably no covalent binding between the second species and the recognition molecule.

The method as described in the fourth aspect of the invention results in an immobilisation of the recognition molecule on the self-assembling monolayer which is high. This means that there are a lot of recognition sites such that the recognition can be higher and the detection limit can be lower. The value of the immobilisation degree depends on the recognition molecule.

For example, the immobilisation degree for proteins is preferably higher than 3500 pg/mm², even more preferably higher than 4000 pg/mm². The high degree of immobilisation of the recognition molecule could be obtained because the succinimide terminated species is directly deposited on the substrate and thereafter, the amino groups of the recognition molecule can directly be coupled to the succiminde groups without an extra activation step.

Introducing an extra activation step (as mentioned in the prior art) lowers the yield of immobilisation degree.

A mixed monolayer is desired as it results in a better sensitivity of the recognition molecule to the target molecule in the medium.

### Experimental results

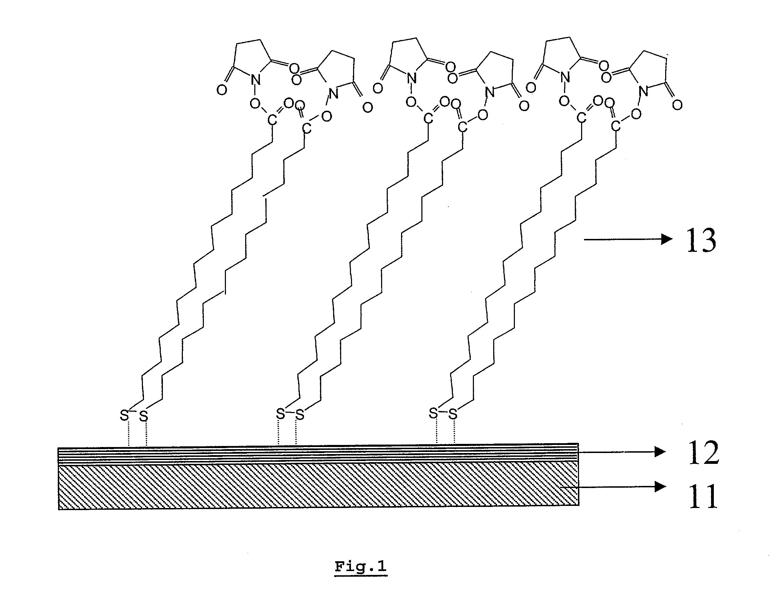

16,16'-Dithiohexadecanoic acid di(N-hydroxysuccinimide ester) (for the purpose of this invention hereafter called DSH SAM) is deposited on a gold layer (12) of a substrate (11). The deposited molecule (13) is represented in figure 1.

The molecule is deposited from a water-free organic solvent like for example THF. The metal substrates are deposited in this solution and the optimal time (at least 3h) is used to organise the thiols into a self-assembled monolayer (SAM). Afterwards the substrate with the SAM is rinsed with THF and dried with nitrogen. Next step is putting this substrate in a solution with the recognition molecules. The recognition molecules will covalently bind without any activation step.

The synthesis of the molecule is:

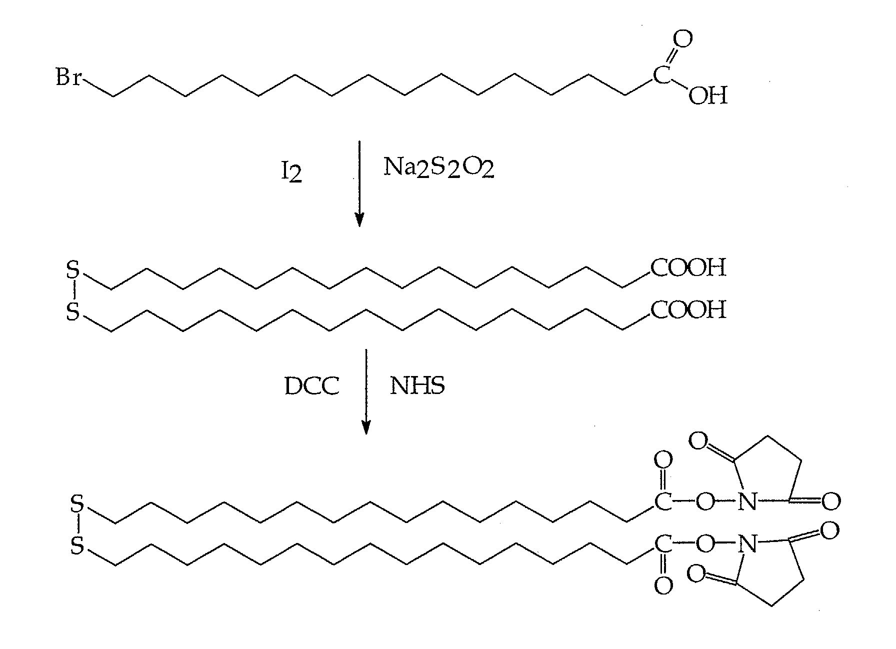

The contact angle of this molecule on a gold surface is 43 ± 1 ° . This is rather hydrophilic, which gives an indication that this surface is suitable for the immobilisation of receptor proteins and that the qualities against non-specific interactions are normally acceptable.

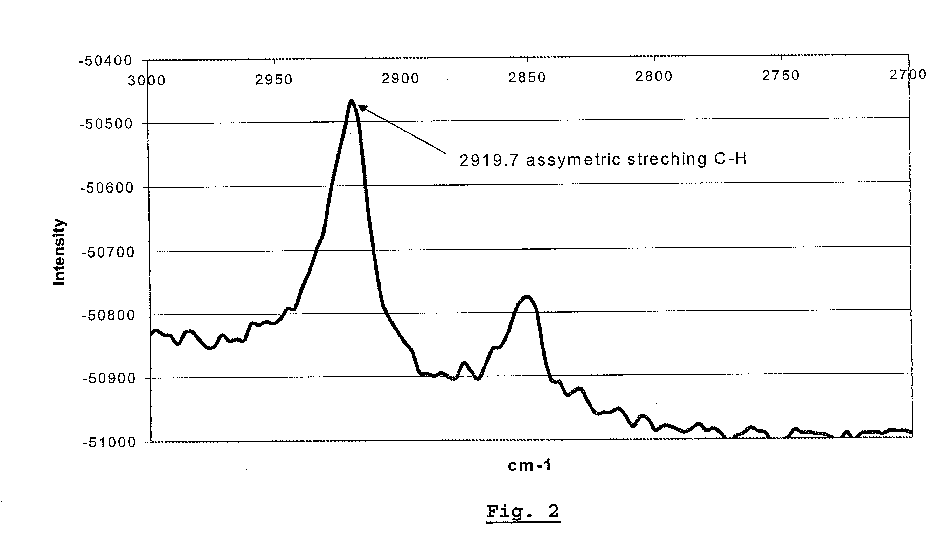

With Grazing Angle FTIR, we reveal that the packing of this molecule on gold is rather good despite the bulky groups and the disulfide bounds (see figure 2).

The asymmetric stretching at 2919.7 gives an indication on the packing of the monolayer. A perfect crystalline like monolayer has this peak at 2918 cm⁻¹ while spaghetti like structure would be around 2925 cm⁻¹. This monolayer can therefore be assigned as a rather well packed monolayer.

For DSH SAM, the deposited monolayer is well formed and can be used to attach antibodies on the surface. No activation step is necessary. The yield is much higher than on a normal thiol.

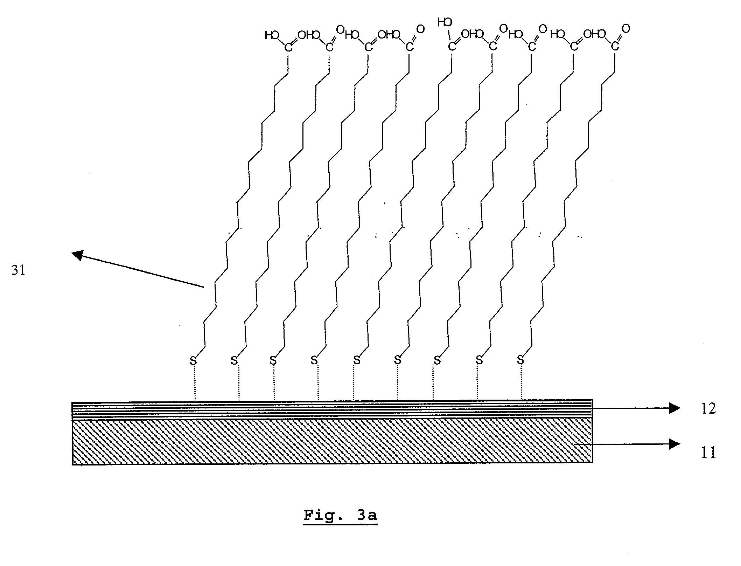

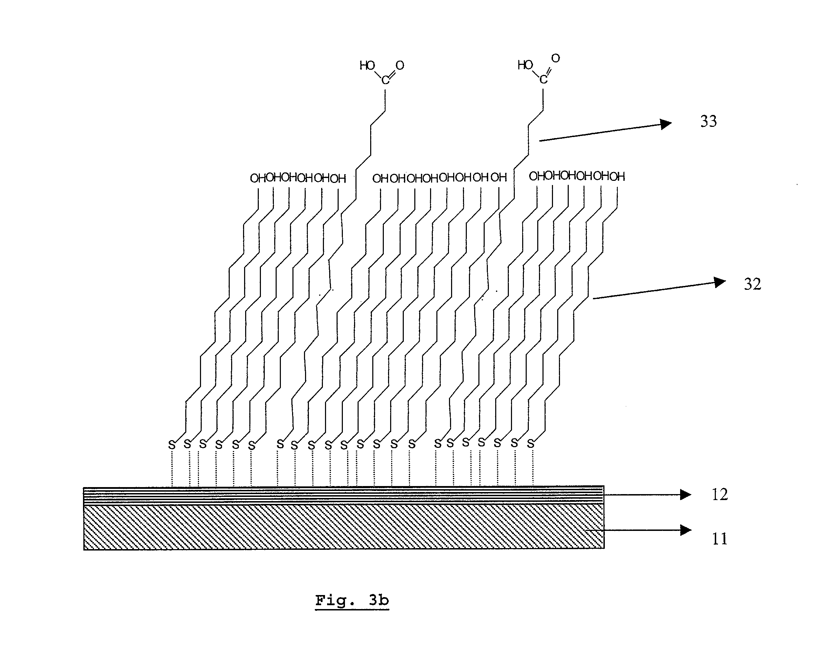

Comparative test between the molecule represented in figure 1 and the molecule represented in figure 3a-c are performed wrt to the immobilisation of antibodies on the molecules represented in figure 3a-c.

Figure 3a: a self-assembling monolayer of molecules 31 (deposited on a substrate 11 having a gold layer 12) is converted to a self-assembling monolayer comprising a N-hydroxysuccinimidyl (NHS) ester from the carboxylic acid groups of the self-assembling monolayer. In a next step, the ester is coupled to the free amine group of the recognition molecule (anti-human transferrine or anti-HT). The immobilization degree is 130 ng/cm2 - 200 ng/cm2.

Figure 3b: a mixed self-assembling monolayer of molecules 32 and 33 is formed on a substrate 11 having a gold layer 12. The carboxylic acid groups (33) of the self-assembling monolayer are converted to a self-assembling monolayer comprising a N-hydroxysuccinimidyl (NHS) ester. In a next step, the ester is coupled to the free amine group of the recognition molecule (anti-human transferrine). The immobilisation degree is 130 ng/cm2 - 250 ng/cm2.

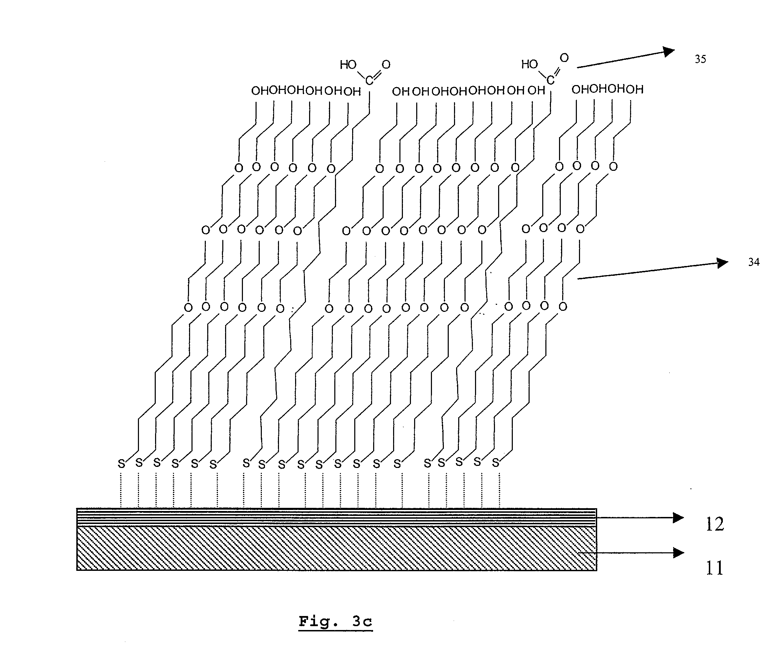

Figure 3c: A mixed Self-assembling monolayer of molecules 34 and 35 is formed on a substrate 11 having a gold layer 12. The self-assembling monolayer comprising carboxylic acid groups (35) is converted to a self-assembling monolayer comprising a N-hydroxysuccinimidyl (NHS) ester. In a next step, the ester is coupled to the free amine group of the recognition molecule (anti-human transferrine). The immobilization degree 130 ng/cm2 - 200 ng/cm2.

The immobilisation of antibodies on DSH SAM (as represented in figure 1) resulted in > 4383 ± 13 pg/mm². It is clear that the immobilisation degree is lower for the molecules represented in figure 3a-c compared to the molecule represented in figure 1.

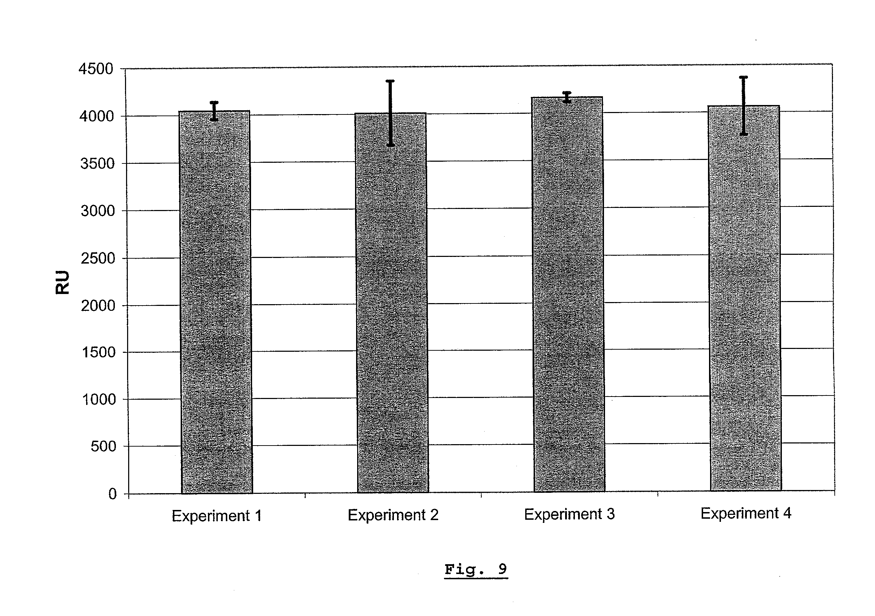

Anti-human Transferrin is immobilized on the DSH SAM, but different measurements were performed on different times and the solutions were prepared at different times. Figure 9 shows very reproducible anti-HT immobilisation. The difference between the different measurements was less than 3.75 %.

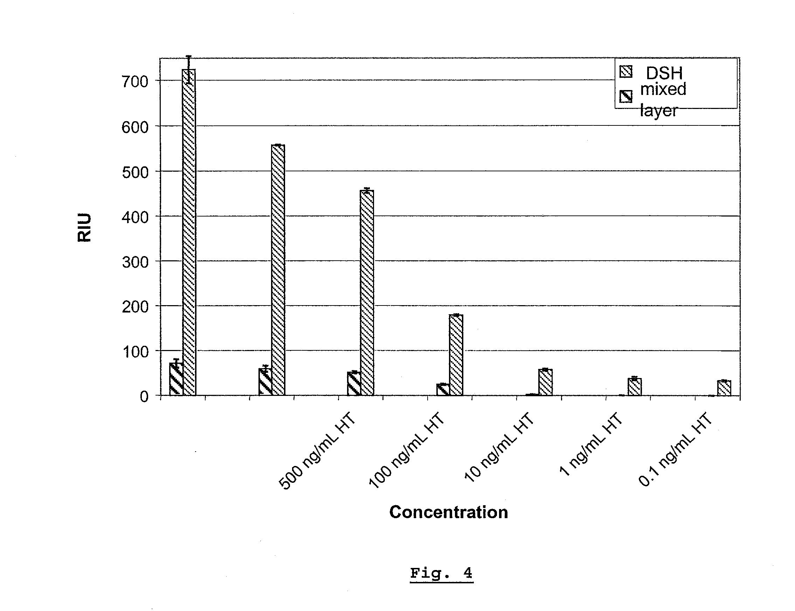

In a further experiment, the recognition of the antigen by the antibody is investigated. In this particular case, we immobilised anti-human transferrine (recognition molecule) and detected human transferrine (target molecule) in different concentrations. This is shown in figure 4. DSH SAM is compared to a mixed monolayer of 16-mercapto-1-hexadecanoic acid and 11-mercapto-1-undecanol, which showed the best results of the monolayers shown in figure 4.

The response is much higher with the DSH SAM compared to a mixed monolayer. In addition, the detection limit is also much lower.

In a next experiment, a mixed monolayer of DSH SAM's and a second species is formed, as shown in figure 5. In a mixed SAM, the DSH acts as the receptor for a recognition molecule, while the other thiols can have groups, which are good against non-specific adsorption like OH, CH₂CH₂OH, CH₂CH₂OCH₃, COOH, ... .

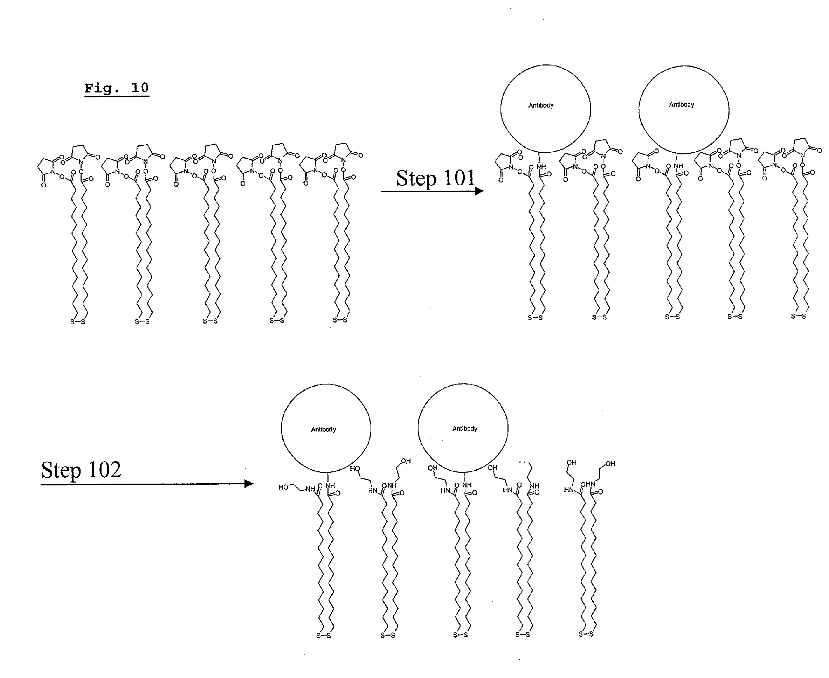

The recognition molecule can directly bind to the N-hydroxysuccinimide group or via a crosslinker to anothergroup than NH₂ of the recognition molecule (step 101). Subsequently, the nonreacted NHS groups should be deactivated so that the analyte (antigen) reacts with the immobilized antibody and not with the surface (step 102). See figure 10. This is performed with ethanolamine. In this experiment, we replaced ethanolamine by a poly(ethyleneoxide) (PEO) containing blocking. The PEO containing blocking agent is : H₂N-(CH₂-CH₂-O)₃-H.

It was observed that there was no difference in antibody immobilisation. There was no difference in recognition of the antigen (Transferrin) (data not shown).

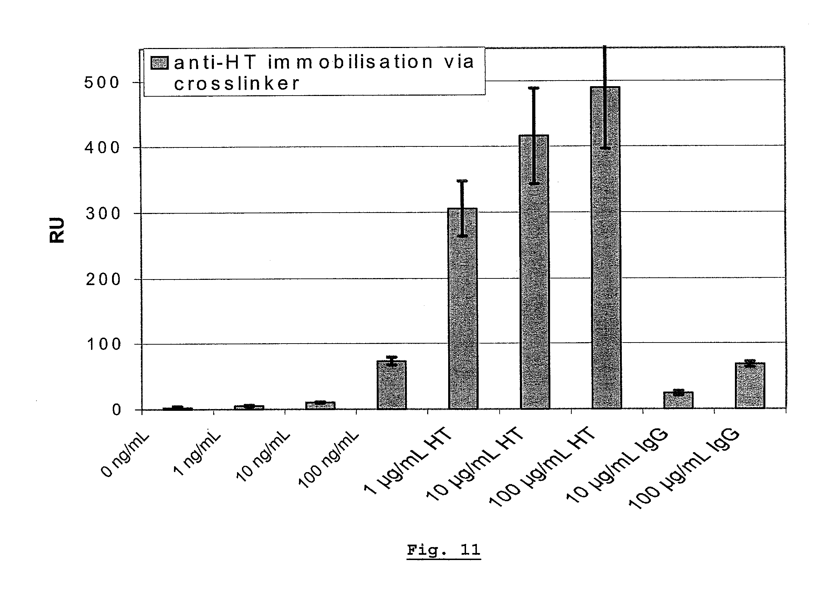

In a further experiment, A versatile surface is realised using an additional crosslinker containing Polyethyeleneoxide (PEO) groups and biotin. After DSH deposition, a NHS terminated surface is achieved. Subsequently, the substrate is subjected to NH₂-polyethyleneoxide-biotin crosslinker . The aminogroup reacts with the NHS groups of the DSH surface. Subsequently, streptavidin is immobilised, followed by biotinylated anti-HT. A surface that is sensitive to for instance biotin-conjugated proteins, DNA or the like is realized. In addition the PEO groups should prevent the non-specific adsorption. The Streptavidin immobilisation on this surface is the recognition of a specific analyte HT and a non-specific analyte IgG are represented in figure 11. It is observed that the recognition of a specific analyte HT is much higher than the recognition of the non-specific analyte IgG.

In another experiment, the molecules as represented in figure 7 (for the purpose of this invention hereafter called PEO SAM) are deposited on a gold layer (12) of a substrate (11).

The synthesis of the molecule is:
The starting molecule (to synthesise the above-mentioned molecule) was synthesised according to J. Lahiri, L.
Isaacs, J. Toe, and G.M. Whitesides, *Analytical Chemistry,* 1999, *71,* 777.

The starting molecule:

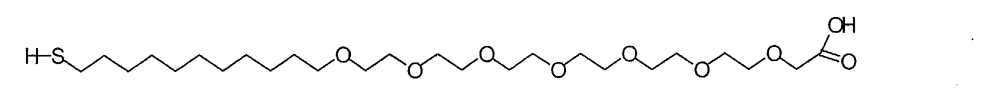

The synthesis route:

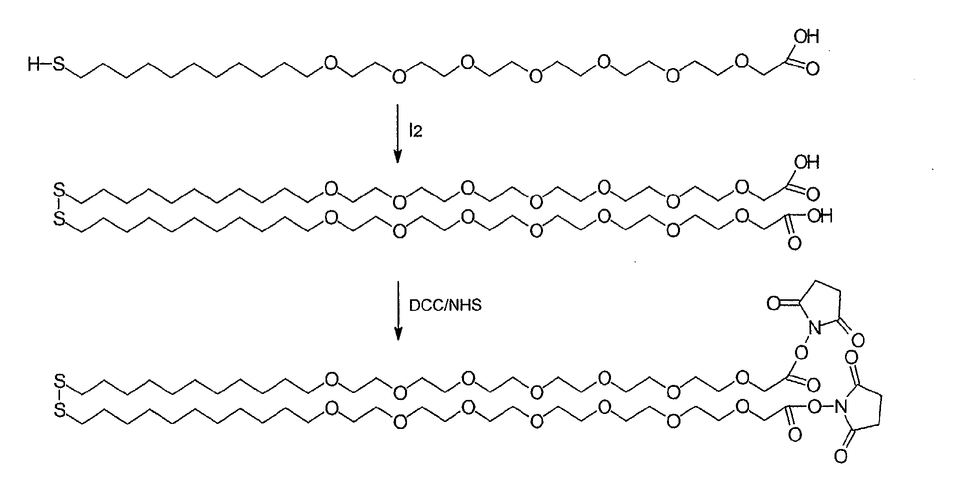

In another embodiment, mixed monolayers are deposited. Therefor, the above mentioned molecules is deposited together with a molecule mentioned in figure 8.

Mixed SAMs can have some additional advantages such as :
Avoiding non-specific adsorption
Avoiding steric hindrance
Enhanced sensitivity

The enhanced qualities of the preactivated DSH SAMs can be used in combination with mixed SAMs.

## Claims

1. Device suitable for the preparation of a sensor, comprising
- a substrate comprising a metal layer, said metal layer comprising at least a first region wherein to said first region is attached a first species having the chemical formula:
X-R₁-S-S-R₂-Y
wherein R₁ and R₂ represent independently from each other a spacer of n carbon atoms, wherein n is an integer higher than 12;
wherein X represents 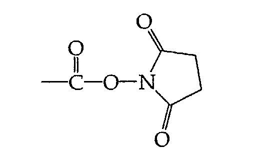 and Y represents an organic group.

2. Device as recited in claim 1 wherein R₁ and R₂ are independently from each other a hydrocarbon chain.

3. A device as recited in claim 2 wherein said hydrocarbon chain is an alkyl chain (CH₂)ₙ.

4. Device as recited in claim 1 wherein R₁ and R₂ are independently from each other Q-R, Q being a hydrocarbon group and Q being bound to the sulphur atom, R being a chemical group for avoiding non-specific adsorption.

5. Device as recited in claim 1 wherein R₁ and R₂ are independently from each other (CH₂)ₐ- (CH₂-CH₂-O)_{b} - (CH₂)_{c}, a being an integer, b being an integer and c being an integer.

6. A device as recited in claim 5 wherein a is comprised between 1 and 20.

7. A device as recited in claim 5 wherein b is comprised between 1 and 10

8. A device as recited in claim 5 wherein c is an integer between 1 and 3.

9. A device as recited in claim 1 to 3, wherein n is an integer comprised between 13 and 30.

10. A device as recited in claim 1 wherein said spacer is interrupted by at least one heteroatom.

11. Device as recited in claim 1 to 10, wherein R₁ and R₂ have the same chemical composition.

12. Device as in claim 1 to 11, wherein Y comprises a chemical group selected from the groups consisting of carboxyl, hydroxyl, cyano, amine, epoxy and vinyl groups.

13. Device as in claim 1 to 11, wherein Y is

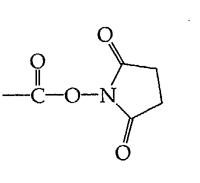

14. Device as in claim 1, wherein said first species is 16,16'-dithiohexadecanoic acid di(N-hydroxysuccinimide ester).

15. Device as recited in claim 1 to 9 wherein said species is

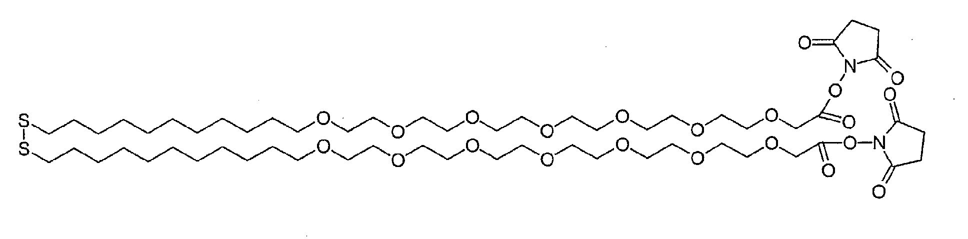

16. A device as recited in claim 1 to 15 wherein said metal layer further comprises a second region, wherein to said second region is attached a second species having the chemical formula:
W-R₃-S-S-R₄-Z,
wherein R₃ and R₄ represent independently from each a spacer, optionally interrupted by a heteroatom, W and Z being organic groups, and wherein said first species and said second species forms a mixed self-assembled monolayer on the metal layer.

17. Device as in claim 15, wherein R₃ and R₄ are a spacer of m carbon atoms, optionally interrupted by q heteroatoms wherein m or (m+q) is an integer higher than 6.

18. Device as recited in claim 15 and 16, wherein W and Z are independently from each other selected from the group consisting of carboxyl, hydroxyl, cyano, amine, epoxy and vinyl groups.

19. Device as in claims 1 to 18, wherein the metal is selected from the groups consisting of gold, silver, mercury, aluminium, platinum, palladium, copper or alloys thereof.

20. Method for producing a device, suitable for determining the presence of a target molecule, comprising the steps of:
- providing a substrate comprising at least a metal layer,
- providing a species having the formula
X-R₁-S-S-R₂-Y
wherein R₁ and R₂ represent independently from each other a spacer of n carbon atoms, wherein n is an integer higher than 12,
wherein X represents 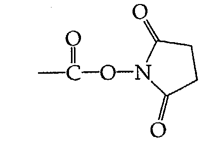 and
wherein Y is an organic group
- and subjecting said substrate to said species to form a self-assembled monolayer on said substrate.

21. A method as recited in claim 19 wherein R₁ and R₂ are independently from each other a hydrocarbon chain.

22. A method as recited in claim 20 wherein said hydrocarbon chain is an alkyl chain (CH₂)ₙ.

23. A method as recited in claim 19 wherein R₁ and R₂ are independently from each other Q-R, Q being a hydrocarbon group and Q being bound to the sulphur atom, R being a chemical group for avoiding non-specific adsorption.

24. A method as recited in claim 19 wherein R₁ and R₂ are independently from each other (CH₂)ₐ - (CH₂-CH₂-O) b-(CH₂)_{c}, a being an integer, b being an integer and c being an integer.

25. A method as recited in claim 19 wherein said spacer is interrupted by at least one heteroatom.

26. Method as recited in claim 19 to 23, further comprising the step of covalently binding a recognition molecule to the X-group of said self-assembling monolayer.

27. Method as in claim 24, wherein said recognition molecule is a chemical compound with a free NH₂ group.

28. Method as in claim 24, wherein said recognition molecule is selected from the group consisting of antigen, antibody, nucleic acid strand, hormones, enzymes, and polyaminoacids.

29. Method as in claim 19 to 26, further comprising the step of :
- providing a second species, said second species being different from said first species,
- subjecting said substrate to said second species to form a mixed self-assembling monolayer is formed on said metal layer.

30. Method as recited in claim 27, wherein said second species has the chemical formula:
W-R₃-S-S-R₄-Z,
wherein R₃ and R₄ represent independently from each other a spacer, optionally interrupted by a heteroatom, W and Z being organic groups.

31. Method as recited in claim 28, wherein W and Z are independently from each other selected from the group consisting of carboxyl, hydroxyl, cyano, amine, epoxy and vinyl groups.

32. Compounds, suitable for forming a monolayer on a sensor device, having the formula:
X-R₁-S-S-R₂-Y
wherein R₁ and R₂ represent independently from each other a spacer of n carbon atoms, wherein n is an integer higher than 12,
wherein X represents 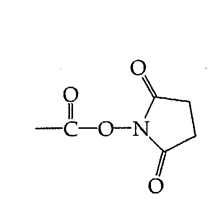 and wherein Y is an organic group.

33. A compound as recited in claim 30 wherein R₁ and R₂ are independently from each other a hydrocarbon chain.

34. A compound as recited in claim 30 wherein said hydrocarbon chain is an alkyl chain (CH₂)ₙ.

35. A compound as recited in claim 30 wherein R₁ and R₂ are independently from each other Q-R, Q being a hydrocarbon group and Q being bound to the sulphur atom, R being a chemical group for avoiding non-specific adsorption.

36. A compound as recited in claim 33 wherein R₁ and R₂ are independently from each other (CH₂)ₐ- (CH₂-CH₂-O)_{b}-(CH₂)_{c}, a being an integer, b being an integer and c being an integer.

37. A compound as recited in claim 34 wherein a is comprised between 1 and 20.

38. A compound as recited in claim 34 wherein b is comprised between 1 and 10

39. A compound as recited in claim 34 wherein c is an integer between 1 and 3.

40. A compound as recited in claim 30 to 37 wherein n is higher than 15.

41. A compound as recited in claim 30 wherein said spacer of n carbon atoms is interrupted by at least one heteroatom.

42. Compound as recited in claim 30 to 39 wherein R₁ and R₂ have the same chemical composition.

43. Compound as in claim 30 to 40, wherein Y is a chemical group selected from the group consisting of carboxyl, hydroxyl, cyano, amine, epoxy and vinyl groups.

44. Compound as in claim 30 to 40, wherein X and Y have the same chemical composition.

45. Compound as in claim 30 wherein said monolayer is 16,16'-dithiohexadecanoic acid di(N-hydroxysuccinimide ester).

46. Compound as recited in claim 30 wherein said species is

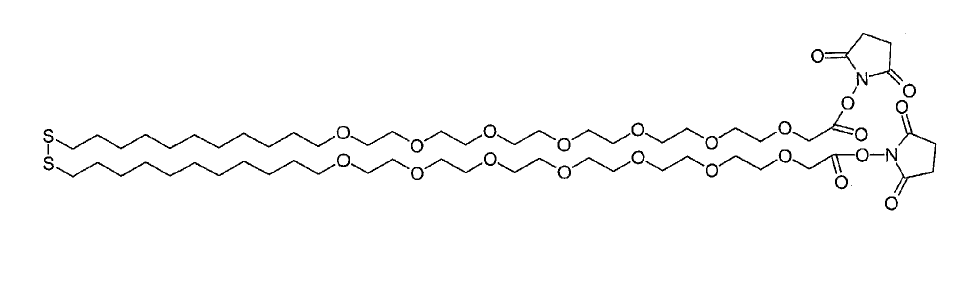

47. Use of a compound as in any of the claims 30 to 44 for the preparation of a self-assembling monolayer on a substrate of a sensor device.
